# EUROPEAN PATENT SPECIFICATION

(11) **EP 3 437 553 B1**
(45) Date of publication and mention of the grant of the patent: **21.01.2026**
(21) Application number: 17775461.1
(22) Date of filing: 30.03.2017
(51) Int. Cl.: A61L 31/02, A61L 31/10, C08K 3/00, C08J 7/04, C08J 5/12, C08J 3/075, A61L 31/00, A61L 31/14, H01B 1/12, A61B 5/25

(54) **COATED COMPOSITE MATERIAL**
BESCHICHTETES VERBUNDMATERIAL
MATÉRIAU COMPOSITE REVÊTU

(30) Priority: 30.03.2016 JP 2016069352
(43) Date of publication of application: 06.02.2019
(73) Proprietor: Tohoku University, Sendai-shi, Miyagi 980-8577 (JP)
(72) Inventor: NISHIZAWA Matsuhiko, Sendai-shi Miyagi 980-8577 (JP); NAGAMINE Kuniaki, Sendai-shi Miyagi 980-8577 (JP); TOMINAGA Teiji, Sendai-shi Miyagi 980-8577 (JP); NAKAZAWA Toru, Sendai-shi Miyagi 980-8577 (JP); IWASAKI Masaki, Sendai-shi Miyagi 980-8577 (JP); NAKAGAWA Atsuhiro, Sendai-shi Miyagi 980-8577 (JP); KAWAGUCHI Tomohiro, Sendai-shi Miyagi 980-8577 (JP); TOKUE Ayako, Sendai-shi Miyagi 980-8577 (JP); NIIKURA Hitoshi, Sendai-shi Miyagi 980-8577 (JP); SAIKI Yoshikatsu, Sendai-shi Miyagi 980-8577 (JP)
(74) Representative: Cohausz & Florack
(86) International application number: PCT/JP2017/013392
(87) International publication number: WO 2017/170927

(56) References cited:
- EP-A1- 1 820 533
- EP-A1- 2 979 726
- WO-A1-00/57948
- WO-A1-2008/150974
- WO-A1-2014/091796
- WO-A1-2014/157550
- TING LING ET AL: "Improvement of drug elution in thin mineralized collagen coatings with PLGA-PEG-PLGA micelles", JOURNAL OF BIOMEDICAL MATERIALS RESEARCH. PART A, 1 January 2013 (2013-01-01), HOBOKEN, NY, US, pages n/a - n/a, XP055633859, ISSN: 1549-3296, DOI: 10.1002/jbm.a.34625

## Description

### TECHNICAL FIELD

The present invention relates to coated composite material. According to the present invention, composite material may be easily delivered to a mount site, and may be mounted.

### BACKGROUND ART

As a method of diagnosis and treatment in the medical field, the measurement of electrical signals emitted by a living body, such as cardio electricity, myoelectricity, or brain electricity, using various devices, and control of biological functions by energization (electrical stimulation) are already generalized. In such methods, an electrode as part of the device is an interface between the device and the living body.

Recently, a composite electrode using an electrode material and hydrogel having excellent biocompatibility as a substrate has been reported (Patent literature 1 and Non-patent literature 1). In such a composite electrode, electrically conducting polymers are electronically polymerized in a condition where the hydrogel (porous body) is placed on the electrode material, and an electrically conducting adhesive layer is formed by extending the electrically conducting polymer from the surface of the electrode material in the vicinity of the electrode material. In such composite electrode, it has been reported that the electrode material and the hydrogel firmly adhere to each other by intertwining polymer chains constituting the hydrogel and the electrically conducting polymer.

### CITATION LIST

### PATENT LITERATURE

[Patent literature 1] WO2014/157550

### NON-PATENT LITERATURE

[Non-patent literature 1] M. Sasaki, et al., Advanced Healthcare Materials, 2014, 3, 1919.

### SUMMARY OF INVENTION

### TECHNICAL PROBLEM

The present inventors have further studied and developed a composite material (composite electrode) in which a substrate (such as electrode material) and a hydrogel (porous body) are bonded with an insulating polymer.

However, since these composite materials have a large volume, it is difficult to deliver and mount the composite materials in the living body.

Therefore, an object of the present invention is to provide a composite material that may be easily delivered and mounted in the living body.

### SOLUTION TO PROBLEM

The present inventors have conducted intensive studies concerning composite material that may be easily delivered and mounted on the living body. As a result, surprisingly found that the following composite material can be used as an electrode or sensor. That is, the volume of the composite material is reduced by drying, and it is coated with the soluble component such as water-soluble compound, temperature-sensitive soluble compound, or pH sensitive soluble compound (for example, a weakly acidic or neutral soluble compound, an acidic soluble compound, or an alkaline soluble compound). The resulting composite material can be delivered to the mounted site in a small volume, and then can be restored to its original volume at the mounted site by dissolving the soluble component. Further, the present inventors found that the following composite material can be used as an electrode or sensor. That is, a volume of the composite material is reduced by folding back the same, and the folded composite material is coated with the soluble component. The resulting composite material can be delivered to the mount site in a small volume, and then can be spread to its original shape at the mount site by dissolving the soluble component.

The present invention is based on the above findings.

The scope of the invention is defined by the claims.

Any references in the description to methods of treatment refer to the products of the present invention for use in a method for treatment.

### ADVANTAGEOUS EFFECTS OF INVENTION

According to the coated composite material of the present invention, the volume of it may be reduced by drying, and it can be delivered to the mount site. Then, the composite material in which the volume is reduced, may be swelled by contacting with moisture, and the volume may be increased. Thus, if the volume increases by contacting with moisture during delivery, it may be difficult to pass narrow areas and the like. However, even if the coated composite material of the present invention contacts with moisture during delivery, it does not swell immediately because of the coating of the soluble component, and thus it is possible to deliver the same to the mount site. Subsequently, after reaching the mount site, the coated composite material may be swelled by addition of solution, heating, addition of weakly acidic or neutral solution, or addition of acidic solution, and can be mounted at the mount site.

Further, the coated composite material contains exothermic particles, and whereby the temperature-sensitive soluble compound may be dissolved by heating, and the composite material may be swelled by moisture. Thus, it can be mounted at mount site. Furthermore, the composite material is folded back, and whereby the volume thereof is reduced and it can be delivered to the mount site. The folded composite material may be delivered to the mount site in a small volume, and then can be spread to its original shape at the mount site by dissolving the soluble component on the surface, to use the same as an electrode or sensor.

### BRIEF DESCRIPTION OF THE DRAWINGS

FIG. 1 are photographs of a dried gel electrode (A) and a coated gel electrode in which the dried gel electrode is coated with gelatin that is a temperature-sensitive soluble compound (B).
FIG. 2 is a graph showing the time of swelling in the case that a dried gel electrode not coated with gelatin, a dried gel electrode coated once with gelatin, and a dried gel electrode coated twice with gelatin were immersed in an artificial cerebrospinal fluid at 37°C.
FIG. 3 are photographs showing the degrees of swelling in the case that the dried gel electrode not coated with gelatin, and the dried gel electrode coated once with gelatin were immersed in an artificial cerebrospinal fluid at 37°C.
FIG. 4 is a graph showing the electrode swelling rate in the case that the coated, dried gel electrode which is coated with gelatin, was heated from 4°C to 37°C (A), and the photographs thereof at 4°C(B) and 37°C (C).
FIG. 5 is a photograph of a coated composite electrode coated with a water-soluble compound, i.e. pectin (A), a graph showing a swelling rate in the case that it was immersed in an artificial cerebrospinal fluid (B), and photographs of a composite electrode without coating (C) and the composite electrode coated with the water-soluble compound, i.e. pectin (D).
FIG. 6 is a photograph of a coated composite electrode coated with a pH sensitive soluble compound, i.e. chitosan, the graph showing a swelling rate in the case where it was immersed in an ARTCEREB (pH7) and a 1% by volume of acetic acid solution (pH4), the photograph of the ARTCEREB-immersed composite electrode (C), and the photograph of the 1% by volume of acetic acid solution immersed composite electrode (D).

### DESCRIPTION OF EMBODIMENTS

### [1] Coated composite material

In the coated composite material of the present invention, a composite material in which a porous body is bound to a substrate, is coated with at least one soluble component selected from a group consisting of a water-soluble compound, a temperature-sensitive soluble compound, and a pH sensitive soluble compound (for example, a weakly acidic or neutral soluble compound, an acidic soluble compound, or an alkaline soluble compound). The coated composite material of the present invention is preferably a coated composite electrode. Further, in the coated composite material of the present invention, the porous body is preferably bound to the substrate by a polymer extending from the substrate to the porous body.

### <<Soluble component>>

The coated composite material of the present invention is coated with the soluble component. As the soluble component, for example, there may be mentioned the water-soluble compound, the temperature-sensitive soluble compound, or the pH sensitive soluble compound (for example, the weakly acidic or neutral soluble compound, the acidic soluble compound, or the alkaline soluble compound).

### (Water-soluble compound)

The water-soluble compound is not particularly limited, so long as the effect of the present invention can be achieved, but includes, for example, starch, polyethylene glycol, glycerogelatin, pullulan, pectin, carrageenan, β-glucan, vinyl ester based polymer, alginic acid, alginic acid derivative, collagen, collagen hydrolyzate, furcellaran, silume seed gum, cassia gum, fenugreek gum, quince seed gum, tarabine gum, almond gum, damson gum, arabinogalactan, dextran, rhamsan gum, levan, azotobacter vinelandii gum, enterobacter gum, welan gum, and a combination of two or more thereof.

By using a water-soluble compound for coating, when the composite material contacts a body fluid in a living body, it is possible to prevent the immediate swelling thereof and suppress the volume of the composite material. The water-soluble compound may be also soluble by body fluids. However, the water-soluble compound does not dissolve due to contact with a small amount of body fluid during delivery. Thus, it is possible to sufficiently suppress the swelling of the composite material during delivery. Further, the water-soluble compound can be dissolved with a mildly irritating aqueous solution such as the body fluid or the phosphate buffer solution, and thus it is advantageous for low irritation to the living body.

### (Temperature-sensitive soluble compound)

The temperature-sensitive soluble compound is not particularly limited, as long as the effect of the present invention can be achieved, but includes, for example gelatin, hard fat, witepsol, cocoa butter, polyvinyl alcohol, and a combination of two or more thereof.

By using the temperature-sensitive soluble compound for coating, it is possible to prevent the immediate swelling thereof and suppress the volume of the composite material. For example, in the case that gelatin, hard fat, witepsol, cocoa butter, or the like which dissolves at about 36°C, is used, it does not dissolve immediately even when it is placed at a temperature of about 36°C in the living body. At a mount site of the composite material, it can be dissolved by a body temperature, and the composite material can be swollen by adding the body fluid or an aqueous solution. That is, the temperature-sensitive soluble compound can be dissolved and the composite material can be swollen without a heating stimulation to a living body.

On the other hand, in the case that polyvinyl alcohol or the like which dissolves at a body temperature or more is used, it does not dissolve at the body temperature (about 36°C) and may be dissolved by heating at the mount site. Thus, it is possible to precisely control the dissolution of the temperature-sensitive soluble compounds and the composite material can be swollen at the mount site by adding the body fluid or an aqueous solution.

### (Exothermic particle)

When the temperature-sensitive soluble compound is used as the soluble component, the temperature-sensitive soluble compound can be dissolved by containing exothermic particles in the coated composite material and heating the porous body. By dissolving the temperature-sensitive soluble compound, moisture can be supplied to the contracted composite material and it can be swollen. The exothermic particles may be contained in the porous body of the coated composite material, or the coated soluble component. Further, they are fixed on surface of the porous body, the substrate, or soluble component. Furthermore, the porous body is preferably not derived from a temperature-responsive polymer, but it is not limited thereto. If the porous body is derived from the temperature-responsive polymer, the porous body sometimes contracts by heating. However, even if the porous body contracts once, the contraction of the porous body is restored by lowering the temperature, and thus the temperature-responsive polymer can be used as the porous body.

The exothermic particle used in the present invention is not particularly limited, so long as the particle can generate heat to specific stimuli, but includes, for example, a magnetic particle generating heat by alternating magnetic fields or microwaves, metal nanoparticle generating heat by light (ultraviolet light, visible light, or infrared light), and carbon nanotubes generating heat by irradiation of near infrared ray.

The magnetic particle is preferably composed of a magnetic material, a ferromagnetic material, an antiferromagnetic material, a ferrimagnetic material, an anti-ferrimagnetic material, or a superparamagnetic material, more preferably composed of iron oxide (in particular superparamagnetic iron oxide), pure iron with an oxide layer, or a material of the general formula M(II)Fe₂O₄ (in which M is Co, Ni, Mn, Zn, Cu, Cd, Ba or other ferrite). The magnetic particles prepared by these materials can be heated by an alternating magnetic field.

For example, the magnetic particle is composed of iron oxide (preferably, magnetite (Fe₃O₄), maghemite (γ-Fe₂O₃), or a mixture of these two oxides), and is preferably superparamagnetic particle. In general, the preferable magnetic particles may be represented by the formula FeO_{X} in which X is a number of 1-2. However, it is also possible to combine magnetic particles with a non-magnetic material such as silicon dioxide (SiO₂). A particle diameter is not particularly limited, as long as the effect of the present invention can be achieved, but preferably 1nm to 1µm, more preferably 2nm to 500nm, even more preferably 5nm to 100nm, most preferably 10nm to 20nm. An average particle diameter is not particularly limited, but preferably 2nm to 300nm, more preferably 5nm to 30nm, even more preferably 10nm to 20nm.

A particle shape is not particularly limited, but spherical particles, rod-like particles, amorphous particles, or the like can be used.

The metal nanoparticle is not particularly limited, but includes, for example, a gold nanoparticle or a silver nanoparticle. A particle diameter of the metal nanoparticle is not particularly limited, as long as the effect of the present invention can be achieved, but preferably 1nm to 1µm, more preferablylnm to 100nm, even more preferably 1nm to 50nm. An average particle diameter of the metal nanoparticle is not particularly limited, but preferably 2nm to 300nm, more preferably 5nm to 30nm, even more preferably 10nm to 20nm.

A particle shape of the metal nanoparticle is not particularly limited, but spherical particles, rod-like particles, amorphous particles, or the like can be used. For example, rod-like gold micro particles in which major axis length is 5 to 100 nm, the minor axis length is 3 to 30 nm, the aspect ratio is 2 to 20, and the absorption maximum wavelength of the localized surface Plasmon resonance is 700 to 2000 nm, can be used.

A content rate of the exothermic particles included in the coated composite material is not particularly limited, so long as the effect of the present invention can be achieved, but preferably 0.001 to 10% by weight, more preferably 0.002 to 5% by weight, further preferably 0.005 to 1% by weight, based on the total amount of the coating composite material and the exothermic particles. When the content rate of the exothermic particles is within the above range, an appropriate calorific value can be obtained and the temperature-sensitive soluble compound can be efficiently dissolved.

### (pH sensitive soluble compound)

The pH sensitive soluble compound used in the present invention is not limited, but includes, the weakly acidic or neutral soluble compound, the acidic soluble compound, and the alkaline soluble compound.

The weakly acidic or neutral soluble compound is not particularly limited, so long as the effect of the present invention can be achieved. However, there may be mentioned, for example, acetyl tributyl citrate, carbomer, cellulose acetate phthalate, cellulose acetate succinate, hypromellose acetate succinate, hypromellose phthalate, polyvinyl acetate phthalate, shellac, tributyl citrate, triethyl citrate, white wax, zein, hydroxypropylmethylcellulose acetate succinate, polyvinyl acetate phthalate, hydroxypropyl methyl cellulose phthalate, wheat gliadin, hypromellose phthalate, and a combination of two or more thereof.

By using the weakly acidic or neutral soluble compound for coating, when the composite material contacts a body fluid in a living body, it is possible to prevent the immediate swelling thereof and suppress the volume of the composite material.

Specifically, by using the weakly acidic soluble compound, it does not dissolve in the body fluid (about pH7.3) in the living body but dissolves at the mount site by adding a weakly acidic solution. Thus, it is possible to precisely control the dissolution of the weakly acidic soluble compounds. Further, the composite material can be immediately swollen at the mount site by adding the body fluid or aqueous solution.

Furthermore, by using the neutral soluble compound, it does not dissolve immediately in the living body. At a mount site of the composite material, it can be dissolved by the body fluid near neutral (about pH 7.3) or an added neutral solution, and the composite material can be swollen. That is, the neutral soluble compound can be dissolved and the composite material can be swollen without a stimulation of pH change to the living body.

The acidic soluble compound is not particularly limited, so long as the effect of the present invention can be achieved, but includes, for example, aminoalkyl methacrylate copolymer E, polyvinyl acetal diethylaminoacetate, chitosan, methacrylic acid copolymer(s), ethylcellulose(s), and a combination of two or more thereof.

When the acidic soluble compound is used for coating, it does not dissolve in the body fluid (about pH7.3) in the living body, and dissolves by adding an acidic solution at the mount site. Thus, it is possible to precisely control the dissolution of the acidic soluble compounds and the composite material can be swollen at the mount site by adding the acidic solution.

The alkaline soluble compound is not particularly limited, so long as the effect of the present invention can be achieved, but includes, for example, ethyl acrylate methyl methacrylate copolymer.

### <<Water content ratio>>

The coated composite material of the present invention is preferably dried one, but it is not limited thereto. The volume of the coated composite material can be reduced by drying. Thus, the water content ratio of the dried coated composite material is not limited, but preferably low. For example, the water content ratio of the dried coated composite material is 20% or less by weight, more preferably 15% or less by weight, even more preferably 10% by weight or less. In connection to this, the water content ratio of the coated composite material which is not dried (that is, the water content when used), is not particularly limited, but preferably 60 to 99.5% by weight, more preferably 70 to 99% by weight, even more preferably 80 to 99% by weight

### <<Drying volume ratio >>

The coated composite material of the present invention is preferably dried one as mentioned above. The volume decreases by drying as compared with the volume of the coated composite material which is freely absorbing moisture. In the present specification, the volume of the dried coated composite material with respect to the volume of the coating composite that freely absorbs moisture is referred to as a drying volume ratio. The drying volume ratio is not particularly limited, but preferably 80% or less, more preferably60% or less, even more preferably 40% or less, most preferably 20% or less. When the drying volume ratio is low, the coated composite material can be delivered to a narrow site in the living body.

### <<Composite material>>

The composite material used in the present invention comprises the substrate and the porous body. In the composite material used in the present invention, the porous body is bound to the substrate by a polymer extending from the substrate to the porous body. The substrate and the porous body can be strongly bonded by bonding with a polymer, but the present invention is not limited thereto. The composite material can be used as a coating composite material by comprising the substrate and the porous body.

As the substrate used in the present invention, an electrically conducting substrate or an insulating substrate can be used.

### (Electrically conducting substrate)

The electrically conducting substrate used in the composite material is not particularly limited as long as it has conductivity, but includes a metal, a carbon material, an electrically conducting polymer material, an elastic material, a semiconductor, or a composite material thereof. When an electrically conducting material is used as the substrate, the composite material of the present invention may be used as a composite electrode.

As the metal, there may be mentioned gold, platinum, silver, titanium, aluminum, tungsten, copper, iron, or palladium. As the carbon material, there may be mentioned carbon nanotube, Ketchen black, glassy carbon, graphene, fullerene, carbon fiber, carbon fabric, or carbon aerogel. As the electrically conducting polymer material, there may be mentioned polyaniline, polyacetylene, polypyrrole, poly (3,4-ethylenedioxythiophene), poly(p-phenylenevinylene), polythiophene, or poly(p-phenylene sulfide). As the elastic material, there may be mentioned urethane, a silicone rubber, or a fluoro-rubber. As the semiconductor, there may be mentioned silicon (Si), germanium, indium tin oxide (ITO), titanium oxide, copper oxide, or silver oxide. They may be used alone or in combination of two or more thereof.

### (Insulating substrate)

As the insulating substrate, glass, general resin or resin composition containing the resin can be used.

As the resin, there may be mentioned polyurethane, polypropylene, polylactic acid, poly(lactide-co-glycolide)copolymer, polydioxanone, acrylonitrile butadiene styrene copolymer, acrylic acid ester, acrylonitrile ethylene propylene rubber styrene copolymer, acrylonitrile styrene copolymer, acrylonitrile styrene acrylate, polybutadiene, bismaleimide triazine, cellulose acetate, cellulose acetate butyrate, cellulose acetate propionate, cyclic butyl terephthalate, cresol formaldehyde, carboxymethyl cellulose, nitrocellulose, hydrin rubber, cellulose propionate, chlorinated vinyl chloride, chloroprene rubber, casein, cellulose triacetate, diallyl phthalate, ethylene chlorotrifluoroethylene copolymer, ethylene diamine tetraacetic acid, ethylene ethyl acrylate, ethylene methyl acrylate, ethylene methacrylic acid, epoxy resin, ethylene propylene diene terpolymer, ethylene tetrafluoroethylene copolymer, ethylene vinyl acetate copolymer, ethyl vinyl ether, perfluoro rubber, polyethylene, polystyrene, butyl rubber, isoprene rubber, diphenylmethane isocyanate, melamine formaldehyde, nitrile rubber, polymethyl methacrylate, polyimide, polyethylene terephthalate, polycarbonate, polyether ether ketone, polyisobutylene, polymethylmethacrylate , polystyrene, polyvinyl acetate, polyvinyl chloride, nylon, polyvinylidene fluoride, polyvinyl pyrrolidone, styrene butadiene, silicone, polyester, Teflon (registered trademark), polytetrafluoroethylene and the like. In particular, polyurethane, polypropylene, polyethylene, polybutadiene, polymethyl methacrylate, polyethylene terephthalate, polyimide, silicone, polyester, Teflon, or polytetrafluoroethylene is preferable from the viewpoint of biocompatibility and stability. They may be used alone or in combination of two or more thereof.

Further, glass is based on silicate as the main component. In the present specification, the silicate includes SiO₂.

When the insulating substrate is used as the substrate, the electrically conducting material is used together with the composite material, to bring the electrically conducting material into contact with the porous body, in order to use the composite material as an electrode or a sensor. The composite material can be used as an electrode or a sensor thereby. As the electrically conducting material, for example, the material used for the electrically conducting substrate can be used without limiting.

### (Porous body)

The porous body contained in the coated composite material of the present invention is not limited as long as it has flexibility, but preferably has an excellent biocompatibility. For example, a gel may be mentioned, and in particular, hydrogel is preferable. The porous body contained in the coated composite material of the present invention is selected from a group consisting of agarose gel, glucomannan gel, polyacrylamide gel, polyacrylamide-2-methylpropanesulfonic acid gel, fibrin gel, polyvinyl alcohol gel, polyhydroxyethyl methacrylate gel, silicone hydrogel, polyvinylpyrrolidone gel, polyethyleneglycol gel, poly(2-acrylamide-2-methylpropanesulfonic acid) gel, alginate gel, carrageenan gel, chitosan gel, poly(N-isopropylacrylamide) gel, acrylic acid gel, polystyrene sulfonic acid gel, PPEGDA, PPEGDM, Polyacrylamide, poly(N, N-dimethylacrylamide), poly (N-isopropylacrylamide), DN hydrogel and a composite gel of two or more thereof.

### (Polymer)

In the substrate and the porous body, the porous body is preferably bound to the substrate by a polymer extending from the substrate to the porous body. The polymer for bonding the substrate and the porous body, is not particularly limited, but includes an electrically conducting polymer and an insulating polymer. The electrically conducting polymer or the insulating polymer extends from the substrate to the inside of the porous body by polymerization so that the substrate and the porous body can be firmly bonded.

The electrically conducting polymer is not particularly limited, but includes poly(3,4-ethylenedioxythiophene) (hereinafter referred to as PEDOT), polyacetylene, polypyrrole, polythiophene, polybithiophene, polyisothiophene, polydodecylthiophene, polyisonaphthothiophene, ploy(3-hexylthiophene), polyaniline, polyisothianaphthene, polythiazyl, polyphenylene, polyfluorene, polydiacetylene, polyacene, polyparaphenylene, polythienylene vinylene, polyphenylenesulfide, or a mixture of two or more thereof, and it is preferably poly(3,4-ethylenedioxythiophene), or polypyrrole. They may be used alone or in combination of two or more thereof.

The insulating polymer is not particularly limited, but includes poly(meth)acrylic acids such as polyacrylic acid, polymethacrylic acid, acrylate/alkyl methacrylate copolymer or salts thereof; synthetic polymers such as polymer of polyethylene glycol dimethacrylate (for example, PPEGDA or PPEGDM), polyhydroxyethylmethacrylate, polyacrylamide, poly(N, N-dimethylacrylamide), poly(2-acrylamido-2-methylpropanesulfonic acid), poly (N-isopropylacrylamide), polyvinylpyrrolidone, poly(styrene sulfonic acid), carboxyvinyl polymer, alkyl modified carboxyvinyl polymer, maleic anhydride copolymer, polyalkylene oxide resin, crosslinked-product of poly(methyl vinyl ether-alt-maleic anhydride) and polyethylene glycol, polyethylene glycol crosslinked-product, N-vinylacetamide crosslinked product, acrylamide crosslinked-product, starch-acrylic acid salt graft copolymer crosslinked-product; and silicone, and, in particular, polyacrylamide, poly (N, N-dimethylacrylamide), PPEGDA and PPEGDM are preferable since deformation due to the surrounding environment is not observed. That is, the swelling rate does not change at temperature or pH. They may be used alone or in combination of two or more thereof.

The composite material may have a wiring for energizing, which extends from the electrically conducting substrate.

The coated composite material of the present invention may be folded back and coated with the soluble component in that state. That is to say, it may be fixed by the soluble component as it is in its folded form. In this embodiment, the coated composite material may be dried. On the other hand, the coated composite material thst is not dried may be preferably used.

### <<Method for preparing composite material>>

The composite material can also be prepared by bonding substrate and porous body. When the porous body is bound to the substrate by a polymer extending from the substrate to the porous body, the composite material may be prepared by the following preparation method (A) or (B). Further, the composite material is preferably a composite electrode, and in the composite material, the porous body is preferably bound to the substrate by the polymer extending from the substrate to the porous body.

### (A) Method for preparing composite material using electrically conducting polymer

When the electrically conducting polymer is used as the polymer, a method for the composite material of the present invention comprises the steps of: (1) forming an electrically conducting substrate on a basal plate for forming substrate, (2) forming a porous body so as to make contact with the electrically conducting substrate formed on the basal plate for forming substrate, (3) binding the electrically conducting substrate and the porous body by electropolymerizing a monomer for an electrically conducting polymer in an electrolyte solution so as to polymerize the electrically conducting polymer from the electrically conducting substrate into the porous body, (4) separating the basal plate for forming substrate from the electrically conducting substrate. Preferably, the method further comprises the step of (5) forming a polymerized layer of an electrically conducting polymer on a surface of the electrically conducting substrate by bringing an electrolyte solution containing a monomer for an electrically conducting polymer into contact therewith and electropolymerizing the monomer for an electrically conducting polymer. The substrate is preferably an electrode, and thus the basal plate for forming substrate is preferably a basal plate for forming electrode.

### (1) Substrate formation step

### (Basal plate for forming substrate)

The basal plate for forming substrate is not limited, so long as an electrically conducting substrate pattern can be formed thereon. As a material of basal plate, there may be mentioned glass, plastic, cloth, or wood. However, the glass is preferable as it is smooth and has a low adhesion to the electrode. Further, a shape of the basal plate for forming substrate is not also limited. The electrically conducting substrate pattern (electrode pattern) may be formed on a platy basal plate, or the electrically conducting substrate pattern (electrode pattern) may be formed on a surface of a rod-shaped substrate.

### (2) Porous body formation step

In the porous body formation step, the porous body is formed as to contact the substrate formed on the basal plate for forming substrate. The porous body may be formed so that the substrate is placed on the porous body, or is buried in the porous body. Further, a preliminarily-formed porous body is arranged as to contact the substrate.

The gelation of the porous body can be performed according to a publicly known method. That is to say, gelation can be performed in accordance with gelation methods of each material.

### (3) Binding step

In the binding step, an adhesion layer of electrically conducting polymer is formed from the electrically conducting substrate to the porous body by electropolymerizing a monomer for electrically conducting polymer to thereby bind the electrically conducting substrate and the porous body. As a monomer for electrically conducting polymer, there may be mentioned 3,4-ethylenedioxythiophene (hereinafter, sometimes referred to as EDOT), acetylene, pyrrole, thiophene, bithiophene, isothiophene, dodecylthiophene, isonaphthothiophene, 3-hexylthiophene, aniline, isothianaphthene, thiazyl, phenylene, fluorene, diacetylene, acene, paraphenylene, thienylene vinylene, phenylenesulfide, or a mixture thereof.

In the binding step, the monomer is dissolved in a solvent, and the solution is added to a gel of a porous body. Then, the electrically conducting polymer is electropolymerized by applying electric potential to the electrically conducting substrate. A concentration of a monomer in the solvent may be appropriately determined. For example, the concentration is 1 to 500mM, preferably 10 to 100mM. The electric potential applied in the electropolymerization may be appropriately determined, but is preferably 0.5 to 1.5V.

### (4) Separation step

In the separation step, the electrically conducting substrate is separated from the basal plate for forming the substrate.

For example, when the Au electrode pattern is formed on the glass basal plate using Au as the substrate (metallic electrode), a binding force between the Au electrode and the porous body is stronger than a binding force between the Au electrode and the glass basal plate. Therefore, the composite material can be separated from the glass basal plate by separating the porous body from the glass basal plate. Further, when a metallic electrode, carbon electrode, or stretch electrode having strong binding force to the glass basal plate is used, for example, it is easily separated by preliminarily applying polyvinyl alcohol on the glass basal plate, and forming the electrode pattern.

### (5) Step for forming electrically conducting polymer-polymerized layer

In the step for forming electrically conducting polymer-polymerized layer, an electrolyte solution containing a monomer for an electrically conducting polymer is brought into contact with the electrically conducting substrate surface, and the electrically conducting polymer-polymerized layer is formed on the electrically conducting substrate surface by electropolymerization.

In this step, the monomer is dissolved in a solvent, and the solution is brought into contact with the electrically conducting substrate surface. Then, the electrically conducting polymer is polymerized on the electrically conducting substrate surface by applying an electric voltage to the electrically conducting substrate.

A concentration of the monomer may be appropriately determined, but for example, the concentration is 1 to 500mM, preferably 10 to 100mM. An amount of solvent with respect to a used volume may be appropriately determined. The applied potential in electropolymerization may be appropriately determined, but preferably 0.5 to 1.5V. As a monomer for electrically conducting polymer used in this step, the electrically conducting polymer used in the above binding step can be used.

### (B) Method for preparing composite material using insulating polymer

The method for preparing the composite material comprises the steps of: (1) introducing a polymerization initiation site on a substrate surface (step for introducing polymerization initiation site), (2) impregnating monomer with a porous body (monomer impregnation step), (3) polymerizing the monomers using the polymerization initiation site as a polymerization initiation point (polymerization step). By the above steps, it is possible to prepare the composite material of the present embodiment having the insulating polymer starting from the surface of the substrate and extending into the porous body. In the method for preparing composite material of this embodiment, the polymerization reaction of the monomer is not particularly limited, but it is preferably a radical polymerization reaction. As the substrate, an insulating substrate, an electrically conducting substrate, or a combination thereof may be used.

### (1) Step for introduction of polymerization initiation site

In this embodiment, the polymerization initiation site is introduced into the substrate surface. As the polymerization initiation site, there may be mentioned an alkylphenone type, an acylphosphine oxide type, an intramolecular hydrogen abstraction type, and other type such as oxime ester (Irgacure (registered trademark) series); a benzoin ether type such as isobutylbenzoin ether, isopropylbenzoin ether; a benzyl ketal type such as benzyl methyl ketal, hydroxycyclohexyl phenyl ketone; a ketone type such as benzophenone, 2-chlorothioxanthone. They may be used alone or in combination of two or more thereof.

In the introduction of the polymerization initiation site, an ozone treatment (introduction of a carboxyl group), an oxygen plasma treatment, a corona discharge treatment, a UV treatment (for example, introduction of benzophenone), a treatment with a silane coupling agent, a treatment with a thiol compound, or the like may be used, and further the polymerization initiation site may be directly introduced by treating the substrate surface. After the above treatments, the polymerization initiation site may be further bound, for example, via an ester bond or an amide bond. Specifically, in the step for introduction of polymerization initiation site, for example, ozone is irradiated to the substrate (for example, polyurethane) placed on a flat plate, to thereby introduce carboxyl groups to the substrate surface, and then α-hydroxyalkylphenone with a hydroxyl group can be bound to the carboxyl groups by using a condensing agent.

Further, for example, benzophenone can be applied to the surface of the substrate (for example, polypropylene) placed on a flat plate, and then the coated substrate can be irradiated with ultraviolet light to thereby introduce benzophenone moieties to the substrate surface.

### (2) Monomer impregnation step

In this embodiment, a monomer is impregnated with the porous body. In this step, as a monomer, a monomer which can be an insulating polymer when it becomes a polymer, is used. Specifically, in the monomer impregnation step, for example, when the porous body is a hydrogel, the hydrogel can be immersed in the aqueous monomer solution.

Further, for example, when the porous body is a polymer hydrogel, the polymer hydrogel is once immersed in water to thereby wash (remove) by-products of the polymerization reaction and contaminated substances such as residual monomers, and then the polymer hydrogel can be immersed in the aqueous monomer solution. As the monomer, a monomer that can be converted into the insulating polymer may be used, or a monomer that can be converted into the conductive polymer may be used.

As the monomer that can be used as the insulating polymer, the monomer constituting the above-mentioned insulating polymer may be used. For example, there may be mentioned (meth)acrylic acids such as acrylic acid, methacrylic acid, acrylate/alkyl methacrylate copolymer or salts thereof; polyethylene glycol dimethacrylate (PEGDA or PEGDM), hydroxyethylmethacrylate, acrylamide, N, N-dimethylacrylamide, 2-acrylamido-2-methylpropanesulfonic acid, N-isopropylacrylamide, vinylpyrrolidone, styrene sulfonic acid, ethylene glycol, maleic anhydride, alkylene oxide, methyl vinyl ether-alt-maleic anhydride, N-vinylacetamide, starch, silanol and the like, and, in particular, acrylamide, N, N-dimethylacrylamide, PEGDA and PEGDM are preferable since deformation due to the surrounding environment is not observed. That is, the swelling rate is unchanged at temperature or pH. They may be used alone or in combination of two or more thereof.

### (3) Polymerization step

Further, in this embodiment, a polymerization reaction of monomers is carried out using the polymerization initiation site as the polymerization initiation point. Examples of the initiation reaction of monomer polymerization include thermal reaction, photoreaction, and oxidation-reduction reaction. Photoreaction is preferable since a reaction control is easy. These reactions may be used alone or in combination of two or more thereof. The conditions for the polymerization initiation reaction can be appropriately determined according to the P characteristics of the polymerization initiation site. The conditions for the polymerization reaction can be appropriately determined according to the properties of the monomer

### (Preparation of composite material containing exothermic particles)

In the "(A) method for preparing composite material using electrically conducting polymer" or the "(B) method for preparing composite material using insulating polymer", the exothermic particles can be contained in the composite material. The portion containing the exothermic particles is not particularly limited, and the exothermic particles can be contained in, for example, the porous body or the substrate. The exothermic particles may be contained in an inside of the porous body of the substrate or may be fixed to a surface of the porous body of the substrate. For example, when the exothermic particles are contained in the porous body, a porous body containing exothermic particles can be prepared by gelatinizing a raw material of a porous body containing exothermic particles. In addition, the exothermic particles can be contained in or adsorbed on the porous body or the substrate by immersing the gelled porous body or the substrate in a liquid in which the exothermic particles are suspended. As the exothermic particles, those described in the above "[1] coated composite material" can be used without limitation.

The porous body can be laminated on the substrate by bringing the raw material of the porous body into contact with the electrically conducting substrate and forming the same. Further, the porous body containing exothermic particles is formed, and then the formed porous body can be laminated to the substrate.

The content of the exothermic particles contained in the raw material of the porous body is not particularly limited as long as the effect of the present invention can be achieved, but preferably 0.001 to 10% by weight, more preferably 0.002 to 5% by weight, even more preferably 0.005 to 1% by weight, based on the total amount of the porous body and the exothermic particles.

### [2] Method for preparing coated composite material

### (2-1) First embodiment of the preparation method

One embodiment of the method for preparing the coated composite material of the present invention **is** as defined in claim 8. composite material used in the preparation method of the present invention, the "composite material" described in the above item "[1] coated composite material" can be used without limitation. The coated composite material obtained by the preparation method of the present invention is preferably the coated composite electrode. Further, in the coated composite material, the porous body is preferably bound to the substrate by the polymer extending from the substrate to the porous body.

### <<Drying step (1)>>

In the drying step of the method for preparing the coated composite material of the present invention, the composite material is dried or dehydrated and the volume thereof is reduced.

The drying method is not particularly limited as long as moisture is removed from the composite material and the volume thereof can be reduced. For example, there may be mentioned a natural drying, a heat drying, a hot air drying, a low-temperature drying, (vacuum) freeze drying, or a drying under pressure, but the natural drying and the heat drying is preferably used. Further, there is a method of dehydrating by immersing the composite material in an ethanol solution.

For example, the heat drying can be carried out as follows. The dry composite material can be obtained by placing the composite electrode in an oven at room temperature to 100 ° C, preferably 50 to 90°C, more preferably 60 to 90°C for several hours.

### <<Coating step (2)>>

In the coating step, the dried composite material is coated with at least one soluble component selected from a group consisting of a water-soluble compound, a temperature-sensitive soluble compound, a weakly acidic or neutral soluble compound, and an acidic soluble compound. As the water-soluble compound, temperature-sensitive soluble compound, and pH sensitive soluble compound used in the coating step of the present invention, the "water-soluble compound", the "temperature-sensitive soluble compound", and the "pH sensitive soluble compound" described in the above item "[1] coated composite material" can be used without limitation.

The coating method of the soluble component is not particularly limited as long as most of the entire surface of the dried composite material is substantially covered. For example, there may be mentioned dipping method, spraying method, printing method (for example, an ink jet printing method or a screen printing method), injection method, vapor deposition method, or aerosol method.

### (2-2) Second embodiment of preparation method

The other embodiment of the method for preparing the coated composite material of the present invention is as defined in claim 9.

### <<Coating step (1)>>

In the coating step, the at least one soluble component selected from a group consisting of water-soluble compound, temperature-sensitive soluble compound, and pH sensitive soluble compound, is coated to the composite material in which the porous body is bound to the substrate. As the water-soluble compound, temperature-sensitive soluble compound, and pH sensitive soluble compound used in the coating step of the present invention, the "water-soluble compound", the "temperature-sensitive soluble compound", and the "pH sensitive soluble compound" described in the above item "[1] coated composite material" can be used.

The coating method of the soluble component is not particularly limited as long as most of the entire surface of the composite material is substantially covered. For example, there may be mentioned dipping method, spraying method, printing method (for example, an ink jet printing method or a screen printing method), injection method, vapor deposition method, or aerosol method.

### <<Drying step (2)>>

In the coating step, the coated composite material is dried. The drying step can be carried out by the same procedure as the drying step of the first embodiment of the preparation method.

### (2-3) Third embodiment of preparation method

Another embodiment of the method preparing the coated composite material of the present invention is as defined in claim 10.

### <<Folding step (1)>>

In the folding step, the composite material in which the porous body is bound to the substrate is folded back so as to reduce a volume. The folding method is not particularly limited, so long as the volume may be reduced. Further, in the folding step, the composite material may be dried before being folded. Furthermore, the composite material may be dried after being folded.

### <<Coating step (2)>>

In the coating step, the at least one soluble component selected from a group consisting of water-soluble compound, temperature-sensitive soluble compound, weakly acidic or neutral soluble compound, and acidic soluble compound, is coated to the folded composite material. By coating the folded composite material with the soluble component, the composite material in the folded state can be fixed by the soluble component. As the water-soluble compound, temperature-sensitive soluble compound, and pH sensitive soluble compound used in the coating step of the present invention, the "water-soluble compound", the "temperature-sensitive soluble compound", and the "pH sensitive soluble compound" described in the above item "[1] coated composite material" can be used. The coating method is not particularly limited, for example, there may be mentioned dipping method, spraying method, printing method (for example, an ink jet printing method or a screen printing method), injection method, vapor deposition method, or aerosol method.

In the coating step of the method for preparing the coated composite material of the present invention, the exothermic particles can be contained in a coating material (soluble component). The exothermic particles may be contained in an inside of the coating material (soluble component) or may be fixed on a surface of the coating material (soluble component). For example, the exothermic particles are suspended in a solution containing the soluble component, and the composite material may be coated with the suspension by dipping method, spraying method, printing method (for example, the ink jet printing method or the screen printing method), injection method, vapor deposition method, or aerosol method. Further, the exothermic particles can be contained in or adsorbed on the porous body or the substrate by immersing the coated composite material in a liquid in which the exothermic particles are suspended, after coating the soluble component.

### [3] Method for swelling composite material

The method for swelling the composite material of the present invention comprises the steps of (1) delivering the coated composite material to a mounting site, and (2)(a) dissolving the soluble component by a body fluid or by adding an aqueous solution, to swell the coated composite material, when the soluble component is the water soluble compound, (b) dissolving the soluble component by a body temperature or by heating, to swell the coated composite material, when the soluble component is the temperature-sensitive soluble compound, (c) dissolving the soluble component by adding a weakly acidic or neutral solution, to swell the coated composite material, when the soluble component is the weakly acidic or neutral soluble compound, (d) dissolving the soluble component by adding an acidic solution, to swell the coated composite material, when the soluble component is the acidic soluble compound, or (e) dissolving the soluble component by adding an alkaline solution, to swell the coated composite material, when the soluble component is the alkaline soluble compound.

In the swelling method, the coated composite material is preferably the coated composite electrode. Further, in the coated composite material, the porous body is preferably bound to the substrate by the polymer extending from the substrate to the porous body.

### <<Delivery step (1)>>

In delivery step (1), the coated composite material is delivered to the mounting site. The delivery method is not particularly limited, so long as the coated composite material can be delivered to the mounting site. For example, the coated composite material can be delivered to the mounting site using an endoscope such as an abdominal endoscope or a neuro endoscope. In delivery step (1), the coated composite material is preferably an electrode comprising the coated composite electrode.

### <<Swelling step (2)>>

In swelling step (2), the soluble component coated on the composite material is dissolved, and the composite material absorbs moisture to thereby swell the composite material and restore to the composite material with volume before drying. In swelling step (2), the dissolution method of each soluble component is different depending on the difference in the soluble components (such as the water-soluble compound, the temperature-sensitive soluble compound, the weakly acidic or neutral soluble compound, or the acidic soluble compound) to be used.

### (a) Dissolution of the water-soluble compound

When the soluble component is the water-soluble compound, the water-soluble compound can be dissolved with the body fluid or the aqueous solution. However, in order to quickly dissolve the water-soluble compound, it is preferable to add a sufficient aqueous solution. The aqueous solution can be added by using, for example, a washing and suction tube.

Preferably, the aqueous solution has little irritation to the living body. For example, there may be mentioned physiological saline, Ringer's solution, dextrose solution, phosphate buffer solution, HEPES buffer solution, and surgical washing perfusion solution. Further, the contracted composite material can be quickly swollen and restored to its original volume, by adding these aqueous solutions.

### (b) Dissolution of temperature-sensitive soluble compound

When the soluble component is the temperature-sensitive soluble compound, the soluble component can be dissolved by body temperature or by heating. For example, when the temperature-sensitive soluble compound that is dissolved at body temperature of about 36°C is used, it can be dissolved at body temperature. However, in order to quickly dissolve the temperature-sensitive soluble compound, further heating is preferable. When the temperature-sensitive soluble compound that is dissolved at a temperature higher than body temperature is used, it is necessary to heat the same.

The heating method is not particularly limited, as long as it is not extremely harmful to the human body. For example, there may be mentioned a heating by applying heat from the outside or a heating by energizing the electrode. The heating temperature is not particularly limited. However, for example, the upper limit is preferably 50°C or less, more preferably 45°C or less. The lower limit of the heating temperature is preferably body temperature, i.e. 36°C or more, more preferably 40°C or more.

As for heating by externally applying heat, in particular, there may be mentioned a heating method by applying far infrared rays or an induction heating method by applying a magnetic field to a composite material in which magnetic particles are embedded.

Specifically, heat can be applied from outside the body by using a thermotherapy device. The heat may be applied by using the high-frequency hyper thermic apparatus (INDIVA, or PhysioPrime) as the thermotherapy device.

Specifically, the heating by energizing the electrode can be carried out as follows. Preliminarily, wiring for heating is fashioned on the composite electrode (for example, the resistance may be increased by a serpentine pattern or the like), and then Joule heat is generated by applying direct current and it is heated.

The temperature-sensitive soluble compound is dissolved by body temperature or by heating, and subsequently the composite material can be swollen by bodily fluid. However, it is preferable to add an aqueous solution in order to quickly swell, and whereby the contracted composite material can be quickly swollen and restored to its original volume.

Furthermore, when the soluble component is a temperature-sensitive soluble compound, it can be heated by causing exothermic particles to generate heat.

### (Heating by microwave)

The microwave can dissolve the temperature-sensitive soluble compound by vibrating water molecules in the hydrogel to generate heat, or causing the magnetic particles in the hydrogel to generate heat. The microwave is the shortest wavelength region of radio waves, and generally means radio waves (electromagnetic waves) with wavelengths from 1 m to 100 µm and frequencies from 300 MHz to 3 THz. A circuit using a magnetron, a klystron, a traveling wave tube (TWT), a gyrotron, or a Gunn diode, or the like can be used as an oscillation source of microwave. The water molecules or the magnetic particles can generate heat by microwaves and the temperature-sensitive can be dissolved. That is, the temperature-sensitive soluble compound can be dissolved and the porous body can be swollen.

In microwave generation, for example, a microwave generation probe used for tissue ablation (for example, an electrosurgical unit for ablation manufactured by Alfresa Pharma Co.) can be used.

### (Heating by alternating magnetic fields)

In the magnetic particles, a relaxation of the magnetic moment in the alternating magnetic field is delayed, whereby the magnetic energy is converted into heat and the magnetic particles generate heat. The intensity of the magnetic field amplitude is usually 1 mT or more, preferably 5 mT or more, more preferably 10 mT or more. It is usually 1000 mT or less, preferably 200 mT or less, more preferably 100 mT or less. If the magnetic field is too small, a sufficient effect of heat generation may not be obtained. If the magnetic field is too large, a high frequency magnetic field may adversely affect a normal body. The frequency of the magnetic field is usually 1 kHz or more, preferably 10 kHz or more, more preferably 100 kHz or more. It is usually 1000 kHz or less, preferably 900 kHz or less, more preferably 800 kHz or less. If the frequency is too small, a sufficient effect of heat generation may not be obtained. If the frequency is too large, a high frequency magnetic field may adversely affect a normal body

### (Heating by laser light)

When the porous body contains metal nanoparticles, the metal nanoparticles can be heated by a laser light to dissolve the temperature-sensitive soluble compound. The laser light is absorbed on a surface of the metal nanoparticles and converted into heat. The temperature-sensitive soluble compound can be dissolved by using the converted heat.

For generation of laser light, for example, a fluorescent navigation light source used for endoscopic surgery (fluorescent imaging system manufactured by KARL STORZ Endoskope Co., Ltd.) can be used.

### (c) Dissolution of weakly acidic or neutral soluble compound

When the soluble component is the weakly acidic soluble compound, the soluble component can be dissolved with a weakly acidic solution. For example, the soluble component can be dissolved by adding the weakly acidic solution with pH 5.0 to 6.5. The weakly acidic solution can be added by using, for example, a washing and suction tube.

Preferably, the weakly acidic solution has little irritation to the living body. For example, there may be mentioned citric acid, acetic acid, or MES buffer solution. Further, the contracted composite material can be quickly swollen and restored to its original volume, by adding these weakly acidic solutions.

On the other hand, when the soluble component is the neutral soluble compound, the soluble component can be dissolved with a neutral solution. For example, the soluble component can be dissolved by adding the neutral solution with pH 6.5 to 8.0. The neutral solution can be added, for example, by using a washing and suction tube.

Preferably, the neutral solution has little irritation to the living body. For example, there may be mentioned physiological saline, Ringer's solution, dextrose solution, phosphate buffer solution, HEPES buffer solution, and surgical washing perfusion solution. Further, the contracted composite material can be quickly swollen and restored to its original volume, by adding these neutral solutions.

### (d) Dissolution of acidic soluble compound

When the soluble component is an acidic soluble compound, the soluble component can be dissolved with an acidic solution. For example, the soluble component can be dissolved by adding the acidic solution with pH 1.0 to 5.0. The acidic solution can be added, for example, by using a washing and suction tube.

Preferably, the acidic solution has little irritation to the living body. For example, there may be mentioned a diluted hydrochloric acid. Further, the contracted composite material can be quickly swollen and restored to its original volume, by adding an acidic solution.

### (e) Dissolution of alkaline soluble compound

When the soluble component is an alkaline soluble compound, the soluble component can be dissolved with an alkaline solution. For example, the soluble component can be dissolved by adding the alkaline solution with pH 8.0 to 11.0. The alkaline solution can be added, for example, by using a washing and suction tube. Preferably, alkaline solution has little irritation to the living body. For example, there may be mentioned a sodium carbonate buffer. Further, the contracted composite material can be quickly swollen and restored to its original volume, by adding the alkaline solution.

### EXAMPLES

The present invention now will be further illustrated by, but is by no means limited to, the following Examples.

### <<Example 1>>

In this example, a composite electrode was prepared by using a double network gel, and the resulting composite electrode was dried. Further, a temperature-soluble gelatin, which is a temperature-sensitive soluble compound, was coated thereon to prepare the coated composite electrode.

### (1) Preparation of composite electrode

A glass slide was cut into appropriately sized pieces, and they were ultrasonically washed for 15 minutes using in order, acetone, 86% ethanol-isopropanol, and distilled water, respectively. Then, they were stored in isopropanol. The glass slide was spin-coated with 10 % by weight of a polyvinyl alcohol solution at 1000rpm for 20 seconds. Then, the basal plate was heated at 70°C by an oven, so as to form a polyvinyl alcohol sacrificial layer by evaporating a solvent.

A solution for polymerization was prepared by mixing 2mL of 1-butanol, 0.22mL of 1M EDOT monomer solution, 6.5mLof 1- butanol solution containing 400mM p-toluene sulfonic acid iron(III) (EDOT oxidant, dopant ion), 22.5mL of tetrahydrofuran solution containing 10 % by weight of polyurethane, and 4.17mL of anisole (for evaporation suppression of the solvent). The solution for polymerization was applied to the glass slide in the line configuration (width 5mm, x length 10mm) using a micro injector (EzROBO-Ace ST4040, Iwashita Engineering, Inc.). The glass slide was heated on a hot plate at 100°C for 10 minutes. A conductive urethane pattern was formed by accelerating evaporation of hte solvent and promoting a polymerization reaction.

Two monomer solutions were prepared as follows. 2-acrylamide-2-methylpropanesulfonic acid, N,N'-ethylenebisacrylamide, APS, and 2-oxoglutaric acid (photo polymerization initiator) were added to distilled water to give 1M, 40mM, 0.9mM, and 2mM, respectively, and sufficiently mixed to prepare a monomer solution 1. Acrylamide, N,N'-ethylenebisacrylamide, APS, and 2-oxoglutaric acid were added to distilled water to give 1M, 1mM, 0.4mM, and 0.5mM, respectively, and sufficiently mixed to prepare a monomer solution 2.

A dimetric frame made from a silicone sheet with a thickness of 0.5 mm was placed on the glass slide. The monomer solution 1 was poured into the frame, and it gelated by being illuminated with ultraviolet light (356nm of wavelength and 8W of output) at room temperature for 6 hours while being covered with a glass slide. The resulting gel was immersed into the monomer solution 2, to thereby infiltrate monomer solution 2 into the gel at 4°C for 24 hours, while blocking out light. Thereafter, the monomer solution 2 was polymerized by being illuminated with ultraviolet light (356nm of wavelength and 8W of output) at room temperature for 6 hours, to obtain a double network gel.

The double network gel was placed on the conductive urethane pattern basal plate. EDOT monomer and LiClO₄(dopant ion) were added to distilled water to give 50mM and 100mM respectively so as to prepare a solution for polymerization. The solution for polymerization was added drop wise on the porous body, and an oxidative electropolymerization having 300mC/cm² of a polymerization amount was performed by applying 1.0V (vs. Ag/AgCl) of electric potential to the conductive urethane and the solution for polymerization.

After the PEDOT polymerization was completed, the polyvinyl alcohol sacrificial layer was dissolved by immersing the substrate in distilled water at100°C for 30 minutes. The double network gel was separated from the basal plate to obtain a double network gel basal plate electrode member patterned with conductive urethane.

### (2) Drying step

The gel electrode swollen with distilled water was sandwiched between two nylon meshes, and further sandwiched between slide glasses. Weight was placed upon and dried overnight at 70 °C in an oven (EYELA Co., NDO-700).

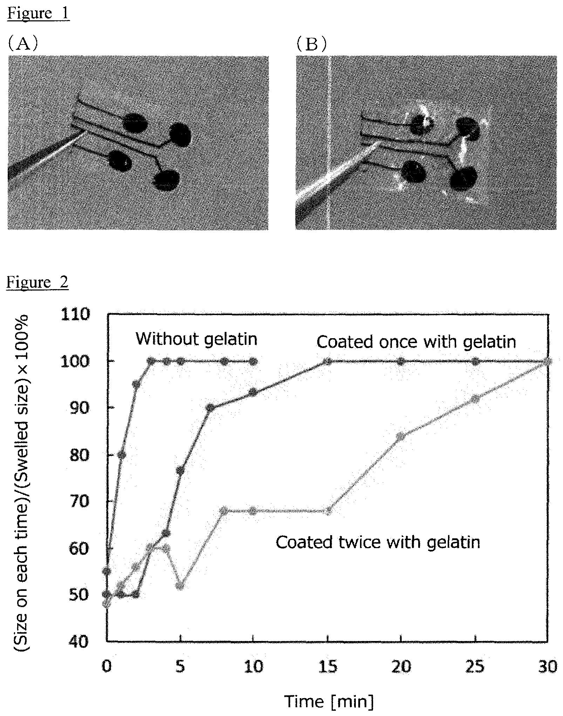

The resulting dried gel electrode is shown in Fig.1A. The volume of the gel electrode was contracted to about 50%.

### (3) Coating step

The resulting dried gel electrode was coated with gelatin. A solution of gelatin (temperature-sensitive soluble compound) was prepared as follows: 6 g of gelatin (Wako Pure Chemical Industries, Ltd.) was added to 30 mL of purified water and stirred to prepare an aqueous gelatin solution (20% by weight). The aqueous gelatin solution was warmed to 50 ° C, and the dried gel electrode was immersed for about 1-2 seconds and recovered. The recovered dried gel electrode was placed in a refrigerator for 10 minutes at 4°C. Gelatin was gelated by cooling in the refrigerator, and the coated gel electrode was dried overnight at room temperature to prepare the coated, dried gel electrode 1 (Fig. 1B). Further, the resulting coated gel electrode 1 was repeatedly coated with gelatin in the same way to prepare the coated, dried gel electrode 2, which was coated with gelatin twice.

### <<Example 2>>

In this Example, a coated composite electrode was prepared using pectin (water-soluble compound) as the soluble component.

The procedure of Example 1 was repeated except that pectin (water-soluble compound) was used instead of the temperature soluble gelatin. Coating step (3) was conducted as follows.

### (3) Coating step

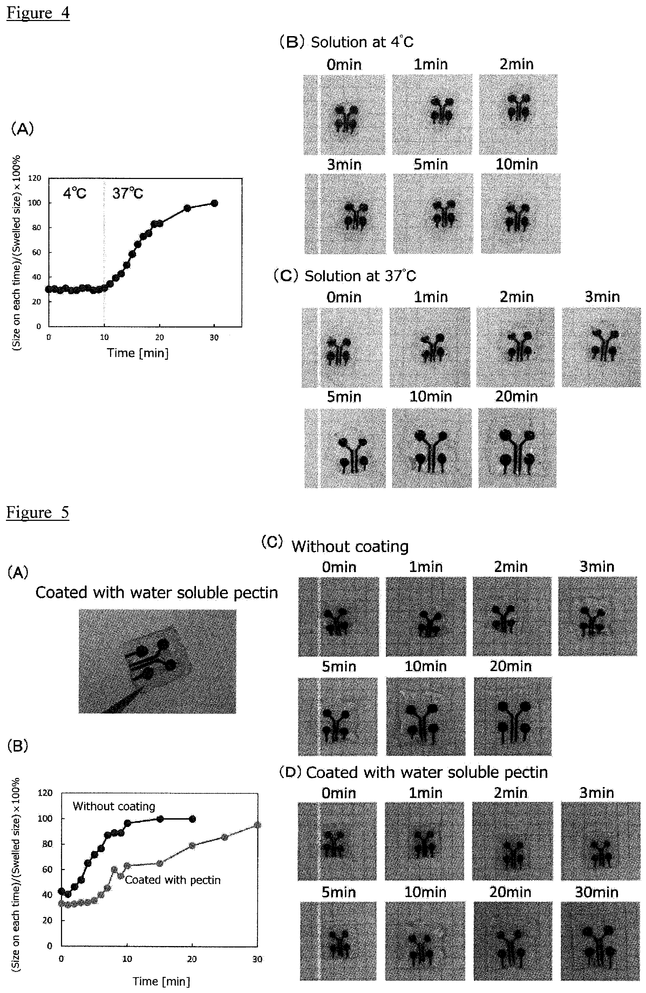

The resulting dried gel electrode was coated with pectin (water-soluble compound). An aqueous solution of pectin (water-soluble compound) was prepared as follows: 1 g of pectin (Wako Pure Chemical Industries, Ltd.) was added to 10 mL of purified water and stirred to prepare an aqueous pectin solution (10% by weight). The dried gel electrode was immersed therein for about 1-2 seconds and recovered. The recovered dried gel electrode was dried overnight at 70 °C in an oven (EYELA Co., NDO-700) to prepare a coated, dried gel electrode (Fig. 5A).

### <<Example 3>>

In this Example, a coated composite electrode was prepared using chitosan (pH sensitive soluble compound) as the soluble component.

The procedure of Example 1 was repeated except that chitosan (pH sensitive soluble compound) was used instead of the temperature soluble gelatin. Coating step (3) was conducted as follows.

### (3) Coating step

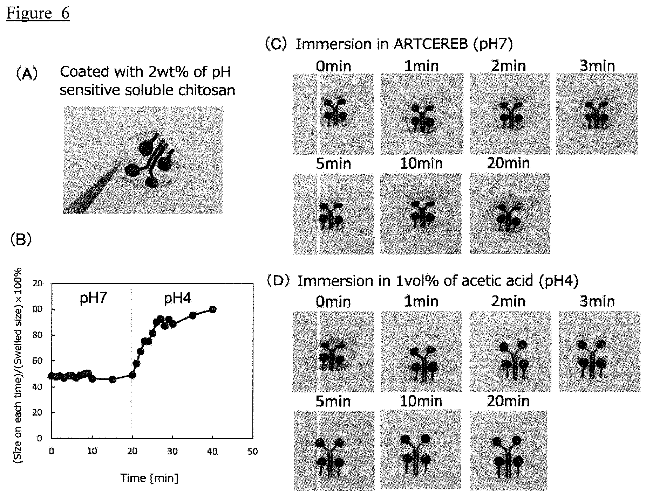

An aqueous solution of chitosan (pH sensitive soluble compound) was prepared as follows: 0.02 g of chitosan (Sigma-Aldrich) was added to 1 mL of acetic acid aqueous solution (1 %vol) and stirred to prepare an aqueous solution (2% by weight). The dried gel electrode was immersed therein for about 1-2 seconds and recovered. The recovered dried gel electrode was dried overnight at 70 °C in an oven to prepare a coated, dried gel electrode (Fig.6A).

### <<Swelling test>>

Swelling tests were conducted on the coated, dried gel electrode 1, which was coated with gelatin once, and the coated, dried gel electrode 2, which was coated with gelatin twice as obtained in Example 1. As a comparative Example, the dried gel electrode, which was not coated with gelatin obtained in the coating step in Example 1, was used.

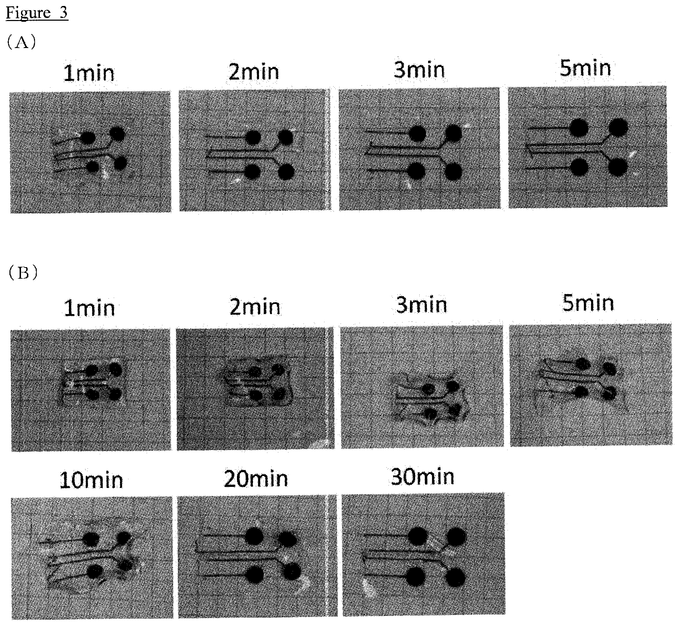

The dried gel electrode (comparative example), the coated, dried gel electrode 1, and the coated, dried gel electrode 2 were immersed in 10 mL of artificial cerebrospinal fluid heated to 37°C and the swelling of the electrodes was measured from 0 to 30 minutes. As shown in Fig.2 and Fig.3A, the dried gel electrode which was not coated with gelatin was restored to its original volume by immersion for 3 minutes. On the other hand, the coated, dried gel electrode 1 was restored in 15 minutes (Fig.2 and Fig.3B), and the coated, dried gel electrode 2 was restored in 30 minutes (Fig.2).

The coated, dried gel electrode 1 obtained in Example 1 was immersed in 10 mL of artificial cerebrospinal fluid cooled to 4°C. After 10 minutes, it was heated to 37°C and the swelling of the electrode was measured. As shown in Fig.4 (A)-(C), it hardly swelled in 10 minutes at 4°C and reverted to original volume in about 20 minutes when heated to 37°C. This result indicated the effect of temperature sensitive coating material.

The swelling test was conducted on the coated, dried gel electrode, which was coated with pectin once as obtained in Example 2. As a comparative Example, the dried gel electrode, which was not coated with pectin, was used. The dried gel electrode (comparative example) and the pectin-coated, dried gel electrode were immersed in 10 mL of artificial cerebrospinal fluid at room temperature, and the swelling of the electrodes was measured from 0 to 30 minutes. As shown in Fig.5(B) and Fig.5(C), the dried gel electrode, which was not coated with pectin was restored to its original volume by the immersion for 3 minutes. On the other hand, the coated, dried gel electrode was restored in 30 minutes.

The swelling test was conducted on the coated, dried gel electrode, which was coated with pH sensitive soluble chitosan as obtained in Example 3. The chitosan-coated, dried gel electrode was immersed in the artificial cerebrospinal fluid (10mL) with pH7, and then, transferred to a 1% by volume of acetic acid aqueous solution (10 mL) with pH 4, and the swelling of the electrode was measured for 20 minutes.

As shown in Fig.3(B)-(D), the chitosan-coated, dried gel electrode scarcely swollen by immersion in artificial cerebrospinal fluid with pH7. However, when it was transferred to the 1% by volume acetic acid aqueous solution with pH4, it was restored to its original volume by immersion for 10 minutes. This result indicated the effect of pH sensitive coating material.

### INDUSTRIAL APPLICABILITY

The coated composite electrode of the present invention may be used for the measurement of electric signals emitted by a living body, such as cardio electricity myoelectricity, or brain electricity, and for the control of biological functions by energization (electrical stimulation).

## Claims

1. A coated composite material **characterized in that** a composite material in which a porous body is bound to a substrate, is coated with at least one soluble component
selected from a group consisting of a water-soluble compound, a temperature-sensitive soluble compound, and a pH sensitive soluble compound,
wherein the porous body is selected from a group consisting of agarose gel, glucomannan gel, polyacrylamide gel, polyacrylamide-2-methylpropanesulfonic acid gel, fibrin gel, polyvinyl alcohol gel, polyhydroxyethyl methacrylate gel, silicone hydrogel, polyvinylpyrrolidone gel, polyethyleneglycol gel, poly(2-acrylamide-2-methylpropanesulfonic acid) gel, alginate gel, carrageenan gel, chitosan gel, poly(N-isopropylacrylamide) gel, acrylic acid gel, polystyrene sulfonic acid gel, PPEGDA, PPEGDM, Polyacrylamide, poly (N, N-dimethylacrylamide), poly (N-isopropylacrylamide), DN hydrogel and a composite gel of two or more thereof and
wherein a water content ratio of the coated composite material is 20% or less by weight or
wherein the coated composite material is folded back.

2. The coated composite material according to claim 1, wherein the pH sensitive soluble compound is a weakly acidic or neutral soluble compound, an acidic soluble compound, or
an alkaline soluble compound.

3. The coated composite material according to a claim 1 or 2, wherein the water-soluble compound is selected from a group consisting of starch, polyethylene glycol, glycerogelatin, pullulan, pectin, carrageenan, β-glucan, vinyl ester based polymer, alginic acid, alginic acid derivative, collagen, collagen hydrolyzate, furcellaran, silume seed gum, cassia gum, fenugreek gum, quince seed gum, tarabine gum, almond gum, damson gum, arabinogalactan, dextran, rhamsan gum, levan, azotobacter vinelandii gum, enterobacter gum, welan gum, and a combination of two or more thereof, the temperature-sensitive soluble compound is selected from a compound consisting a group of gelatin, hard fat, witepsol, cocoa butter, polyvinyl alcohol, and a combination of two or more thereof, the pH sensitive soluble compound is the weakly acidic or neutral soluble compound and selected from a group consisting of acetyl tributyl citrate, carbomer, cellulose acetate phthalate, cellulose acetate succinate, hypromellose acetate succinate, hypromellose phthalate, polyvinyl acetate phthalate, shellac, tributyl citrate, triethyl citrate, white wax, zein, hydroxypropylmethylcellulose acetate succinate, polyvinyl acetate phthalate, hydroxypropyl methyl cellulose phthalate, wheat gliadin, hypromellose phthalate, and a combination of two or more thereof, the pH sensitive soluble compound is the acidic soluble compound and selected from a group consisting of aminoalkyl methacrylate copolymer E(s) , polyvinyl acetal diethylaminoacetate, chitosan, methacrylic acid copolymer(s), ethylcellulose(s), and a combination of two or more thereof, and the pH sensitive soluble compound is the alkaline soluble compound and is ethyl acrylate methyl methacrylate copolymer.

4. The coated composite material according to any one of claims 1 to 3, comprising exothermic particles.

5. The coated composite material according to any one of claims 1 to 4, wherein the composite material is a composite electrode.

6. The coated composite material according to any one of claims 1 to 5, wherein the porous body is bound to the substrate by a polymer extending from the substrate to the porous body.

7. The coated composite material according to claim 6, wherein the polymer is at least one electrically conducting polymer selected from a group consisting of poly(3,4-ethylenedioxythiophene), polyacetylene, polypyrrole, polythiophene, polybithiophene, polyisothiophene, polydodecylthiophene, polyisonaphthothiophene, poly(3-hexylthiophene), polyaniline, polyisothianaphthene, polythiazyl, polyphenylene, polyfluorene, polydiacetylene, polyacene, polyparaphenylene, polythienylene vinylene, and polyphenylenesulfide, or an insulating polymer selected from a group consisting of poly(meth)acrylic acid or a salt thereof (for example, poly(meth)acrylic acids such as polyacrylic acid, polymethacrylic acid, acrylate/alkyl methacrylate copolymer, or a salt thereof), polymer of polyethylene glycol dimethacrylate (for example, PPEGDA or PPEGDM), polyhydroxyethylmethacrylate, polyacrylamide, poly(N, N-dimethylacrylamide), poly(2-acrylamido-2-methylpropanesulfonic acid), poly (N-isopropylacrylamide), polyvinylpyrrolidone, poly(styrene sulfonic acid), carboxyvinyl polymer, alkyl modified carboxyvinyl polymer, maleic anhydride copolymer, polyalkylene oxide resin, crosslinked product of poly(methyl vinyl ether-alt-maleic anhydride) and polyethylene glycol, polyethylene glycol crosslinked product, N-vinylacetamide crosslinked product, acrylamide crosslinked product, starch-acrylic acid salt graft copolymer crosslinked product, and silicone.

8. A method for preparing the coated composite material according to any one of claims 1 to 7, the method comprising the steps of:
(1) drying a composite material in which a porous body is bound to a substrate, and
(2) coating the dried composite material with at least one soluble component selected from a group consisting of a water-soluble compound, a temperature-sensitive soluble compound, and a pH sensitive soluble compound.

9. A method for preparing the coated composite material according to any one of claims 1 to 7, the method comprising the steps of:
(1) coating a composite material in which a porous body is bound to a substrate, with at
least one soluble component selected from a group consisting of a water-soluble compound, a temperature-sensitive soluble compound, and a pH sensitive soluble compound, and
(2) drying the coated composite material.

10. A method for preparing the coated composite material according to any one of claims 1 to 7, the method comprising the steps of:
(1) folding back a composite material in which a porous body is bound to a substrate,
and
(2) coating the folded composite material with at least one soluble component selected from a group consisting of a water-soluble compound, a temperature-sensitive soluble compound, and a pH sensitive soluble compound.

11. The method for preparing a coated composite material according to any one of claims 8 to 10, wherein the coated composite material comprises exothermic particles.

12. The method for preparing a coated composite material according to any one of claims 8 to 11, wherein the water-soluble compound is selected from a group consisting of starch, polyethylene glycol, glycerogelatin, pullulan, pectin, carrageenan, β-glucan, vinyl ester based polymer, alginic acid, alginic acid derivative, collagen, collagen hydrolyzate, furcellaran, silume seed gum, cassia gum, fenugreek gum, quince seed gum, tarabine gum, almond gum, damson gum, arabinogalactan, dextran, rhamsan gum, levan, azotobacter vinelandii gum, enterobacter gum, welan gum, and a combination of two or more thereof, the temperature-sensitive soluble compound is selected from a compound consisting a group of gelatin, hard fat, witepsol, cocoa butter, polyvinyl alcohol, and a combination of two or more thereof, the pH sensitive soluble compound is the weakly acidic or neutral soluble compound and selected from a group consisting of acetyl tributyl citrate, carbomer, cellulose acetate phthalate, cellulose acetate succinate, hypromellose acetate succinate, hypromellose phthalate, polyvinyl acetate phthalate, shellac, tributyl citrate, triethyl citrate, white wax, zein, hydroxypropylmethylcellulose acetate succinate, polyvinyl acetate phthalate, hydroxypropyl methyl cellulose phthalate, wheat gliadin, hypromellose phthalate, and a combination of two or more thereof, the pH sensitive soluble compound is the acidic soluble compound and selected from a group consisting of aminoalkyl methacrylate copolymer E(s) , polyvinyl acetal diethylaminoacetate, chitosan, methacrylic acid copolymer(s), ethylcellulose(s), and a combination of two or more thereof, and the pH sensitive soluble compound is the alkaline soluble compound and is ethyl acrylate methyl methacrylate copolymer.

13. The method for preparing a coated composite material according to any one of claims 8 to 12, wherein the coated composite material is a coated composite electrode.

14. The method for preparing a coated composite material according to any one of claims 8 to 13, wherein the porous body is bound to the substrate by polymerizing monomers from the substrate to the porous body.

## Patentansprüche

1. Ein beschichtetes Verbundmaterial, **dadurch gekennzeichnet, dass** ein Verbundmaterial, in welchem ein poröser Körper an ein Substrat gebunden ist, mit mindestens einer löslichen Komponente beschichtet ist, die aus einer Gruppe ausgewählt ist, die aus einer wasserlöslichen Verbindung, einer temperaturempfindlichen löslichen Verbindung und einer pH-empfindlichen löslichen Verbindung besteht, wobei der poröse Körper aus einer Gruppe ausgewählt ist, die aus Agarosegel, Glucomannangel, Polyacrylamidgel, Polyacrylamid-2-methylpropansulfonsäuregel, Fibringel, Polyvinylalkoholgel, Polyhydroxyethylmethacrylatgel, Siliconhydrogel, Polyvinylpyrrolidongel, Polyethylenglykolgel, Poly(2-acrylamid-2-methylpropansulfonsäure)gel, Alginatgel, Carrageenangel, Chitosangel, Poly(N-isopropylacrylamid)gel, Acrylsäuregel, Polystyrolsulfonsäuregel, PPEGDA, PPEGDM, Polyacrylamid, Poly(N,N-dimethylacrylamid), Poly(N-isopropylacrylamid), DN-Hydrogel und einem Kompositgel aus zweien oder mehr davon besteht, und wobei der Wassergehalt des beschichteten Verbundmaterials 20 Gew.-% oder weniger beträgt oder wobei das beschichtete Verbundmaterial zurückgefaltet ist.

2. Das beschichtete Verbundmaterial nach Anspruch 1, wobei die pH-empfindliche lösliche Verbindung eine schwach saure oder neutrale lösliche Verbindung, eine säurelösliche Verbindung oder eine alkalisch lösliche Verbindung ist.

3. Das beschichtete Verbundmaterial nach Anspruch 1 oder 2, wobei die wasserlösliche Verbindung aus einer Gruppe ausgewählt ist, die aus Stärke, Polyethylenglykol, Glycerogelatin, Pullulan, Pektin, Carrageenan, β-Glucan, Vinylester-basiertem Polymer, Alginsäure, Alginsäurederivat, Collagen, Collagenhydrolysat, Furcellaran, Silumesamengummi, Cassiagummi, Bockshornkleegummi, Quittensamengummi, Tarabingummi, Mandelgummi, Zwetschgengummi, Arabinogalactan, Dextran, Rhamsangummi, Levan, Azotobacter-vinelandii-Gummi, Enterobacter-Gummi, Welan-Gummi und einer Kombination aus zweien oder mehr davon besteht, die temperaturempfindliche lösliche Verbindung aus einer Verbindung ausgewählt ist, die aus einer Gruppe aus Gelatine, Hartfett, Witepsol, Kakaobutter, Polyvinylalkohol und einer Kombination aus zweien oder mehr davon besteht, die pH-empfindliche lösliche Verbindung die schwach saure oder neutrale lösliche Verbindung ist und aus einer Gruppe ausgewählt ist, die aus Acetyltributylcitrat, Carbomer, Celluloseacetatphthalat, Celluloseacetatsuccinat, Hypromelloseacetatsuccinat, Hypromellosephthalat, Polyvinylacetatphthalat, Schellack, Tributylcitrat, Triethylcitrat, weißem Wachs, Zein, Hydroxypropylmethylcelluloseacetatsuccinat, Polyvinylacetatphthalat, Hydroxypropylmethylcellulosephthalat, Weizengliadin, Hypromellosephthalat und einer Kombination aus zweien oder mehr davon besteht, die pH-empfindliche lösliche Verbindung die säurelösliche Verbindung ist und aus einer Gruppe ausgewählt ist, die aus Aminoalkylmethacrylat-Copolymer(en) E, Polyvinylacetaldiethylaminoacetat, Chitosan, Methacrylsäure-Copolymer(en), Ethylcellulose(n) und einer Kombination aus zweien oder mehr davon besteht, und die pH-empfindliche lösliche Verbindung die alkalisch lösliche Verbindung ist und ein Ethylacrylatmethylmethacrylat-Copolymer ist.

4. Das beschichtete Verbundmaterial nach einem der Ansprüche 1 bis 3, welches exotherme Partikel umfasst.

5. Das beschichtete Verbundmaterial nach einem der Ansprüche 1 bis 4, wobei das Verbundmaterial eine Verbundelektrode ist.

6. Das beschichtete Verbundmaterial nach einem der Ansprüche 1 bis 5, wobei der poröse Körper an das Substrat durch ein Polymer gebunden ist, das sich von dem Substrat bis an den porösen Körper erstreckt.

7. Das beschichtete Verbundmaterial nach Anspruch 6, wobei das Polymer mindestens ein elektrisch leitendes Polymer ist, das aus einer Gruppe ausgewählt ist, die aus Poly(3,4-ethylendioxythiophen), Polyacetylen, Polypyrrol, Polythiophen, Polybithiophen, Polyisothiophen, Polydodecylthiophen, Polyisonaphthothiophen, Poly(3-hexylthiophen), Polyanilin, Polyisothianaphthen, Polythiazyl, Polyphenylen, Polyfluoren, Polydiacetylen, Polyacen, Polyparaphenylen, Polythienylenvinylen und Polyphenylensulfid besteht, oder ein isolierendes Polymer ist, das aus einer Gruppe ausgewählt ist, die aus Poly(meth)acrylsäure oder einem Salz davon (beispielsweise Poly(meth)acrylsäure wie Polyacrylsäure, Polymethacrylsäure, Acrylat/Alkylmethacrylat-Copolymer oder einem Salz davon), Polymer von Polyethylenglykoldimethacrylat (beispielsweise PPEGDA oder PPEGDM), Polyhydroxyethylmethacrylat, Polyacrylamid, Poly(N,N-dimethylacrylamid), Poly(2-acrylamido-2-methylpropansulfonsäure), Poly(N-isopropylacrylamid), Polyvinylpyrrolidon, Poly(styrolsulfonsäure), Carboxyvinylpolymer, Alkyl-modifiziertem Carboxyvinylpolymer, Maleinsäureanhydrid-Copolymer, Polyalkylenoxidharz, vernetztem Produkt von Poly (methylvinylether-alt-maleinsäureanhydrid) und Polyethylenglykol, Polyethylenglykol-vernetztem Produkt, N-Vinylacetamid-vernetztem Produkt, Acrylamid-vernetztem Produkt, Stärke-Acrylsäuresalz-Pfropfcopolymer-vernetztem Produkt und Silicon besteht.

8. Ein Verfahren zur Herstellung des beschichteten Verbundmaterials nach einem der Ansprüche 1 bis 7, umfassend die Schritte:
(1) Trocknen eines Verbundmaterials, bei welchem ein poröser Körper an ein Substrat gebunden ist, und
(2) Beschichten des getrockneten Verbundmaterials mit mindestens einer löslichen Komponente, die aus einer Gruppe ausgewählt ist, die aus einer wasserlöslichen Verbindung, einer temperaturempfindlichen löslichen Verbindung und einer pH-empfindlichen löslichen Verbindung besteht.

9. Ein Verfahren zur Herstellung des beschichteten Verbundmaterials nach einem der Ansprüche 1 bis 7, umfassend die Schritte:
(1) Beschichten eines Verbundmaterials, bei welchem ein poröser Körper an ein Substrat gebunden ist, mit mindestens einer löslichen Komponente, die aus einer Gruppe ausgewählt wird, die aus einer wasserlöslichen Verbindung, einer temperaturempfindlichen löslichen Verbindung und einer pH-empfindlichen löslichen Verbindung besteht, und
(2) Trocknen des beschichteten Verbundmaterials.

10. Ein Verfahren zur Herstellung des beschichteten Verbundmaterials nach einem der Ansprüche 1 bis 7, umfassend die Schritte:
(1) Zurückfalten eines Verbundmaterials bei welchem ein poröser Körper an ein Substrat gebunden ist, und
(2) Beschichten des gefalteten Verbundmaterials mit mindestens einer löslichen Komponente, die aus einer Gruppe ausgewählt ist, die aus einer wasserlöslichen Verbindung, einer temperaturempfindlichen löslichen Verbindung und einer pH-empfindlichen löslichen Verbindung besteht.

11. Das Verfahren zur Herstellung eines beschichteten Verbundmaterials nach einem der Ansprüche 8 bis 10, wobei das beschichtete Verbundmaterial exotherme Partikel umfasst.

12. Das Verfahren zur Herstellung eines beschichteten Verbundmaterials nach einem der Ansprüche 8 bis 11, wobei die wasserlösliche Verbindung aus einer Gruppe ausgewählt ist, die aus Stärke, Polyethylenglykol, Glycerogelatin, Pullulan, Pektin, Carrageenan, β-Glucan, Vinylester-basiertem Polymer, Alginsäure, Alginsäurderivat, Collagen, Collagenhydrolysat, Furcellaran, Silumesamengummi, Cassiagummi, Bockshornkleegummi, Quittensamengummi, Tarabingummi, Mandelgummi, Zwetschgengummi, Arabinogalactan, Dextran, Rhamsangummi, Levan, Azotobacter-vinelandii-Gummi, Enterobacter-Gummi, Welan Gummi und einer Kombination aus zweien oder mehr davon besteht, die temperaturempfindliche lösliche Verbindung aus einer Verbindung ausgewählt ist, die aus einer Gruppe aus Gelatine, Hartfett, Witepsol, Kakaobutter, Polyvinylalkohol und einer Kombination aus zweien oder mehr davon besteht, die pH-empfindliche lösliche Verbindung die schwach saure oder neutrale lösliche Verbindung ist und aus einer Gruppe ausgewählt ist, die aus Acetyltributylcitrat, Carbomer, Celluloseacetatphthalat, Celluloseacetatsuccinat, Hypromelloseacetatesuccinat, Hypromellosephthalat, Polyvinylacetatphthalat, Schellack, Tributylcitrat, Triethylcitrat, weißem Wachs, Zein, Hydroxypropylmethylcelluloseacetatsuccinat, Polyvinylacetatphthalat, Hydroxypropylmethylcellulosephthalat, Weizengliadin,
Hypromellosephthalat und einer Kombination aus zweien oder mehr davon besteht, die pH-empfindliche lösliche Verbindung die säurelösliche Verbindung ist und aus einer Gruppe ausgewählt ist, die aus Aminoalkylmethacrylat-Copolymer(en) E, Polyvinylacetaldiethylaminoacetat, Chitosan, Methacrylsäure-Copolymer(en), Ethylcellulose(n) und einer Kombination aus zweien oder mehr davon besteht, und die pH-empfindliche lösliche Verbindung die alkalisch lösliche Verbindung ist und Ethylacrylatmethylmethacrylat-Copolymer ist.

13. Das Verfahren zur Herstellung eines beschichteten Verbundmaterials nach einem der Ansprüche 8 bis 12, wobei das beschichtete Verbundmaterial eine beschichtete Verbundelektrode ist.

14. Das Verfahren zur Herstellung eines beschichteten Verbundmaterials nach einem der Ansprüche 8 bis 13, wobei der poröse Körper an das Substrat durch Polymerisieren von Monomeren von dem Substrat zu dem porösen Körper gebunden wird.

## Revendications

1. Matériau composite revêtu **caractérisé en ce qu'un** matériau composite, dans lequel un corps poreux est lié à un substrat, est revêtu d'au moins un composant soluble choisi dans un groupe composé d'un composé soluble dans l'eau, d'un composé soluble sensible à la température et d'un composé soluble sensible au pH,
dans lequel le corps poreux est choisi dans un groupe constitué d'un gel d'agarose, d'un gel de glucomannane, d'un gel de polyacrylamide, d'un gel de polyacrylamide-2-méthylpropanesulfonique, d'un gel de fibrine, d'un gel d'alcool polyvinylique, d'un gel de méthacrylate de polyhydroxyéthyle, d'un hydrogel de silicone, d'un gel de polyvinylpyrrolidone, d'un gel de polyéthylèneglycol, d'un gel de poly(2-acrylamide-2-méthylpropanesulfonique), d'un gel d'alginate, d'un gel de carraghénane, d'un gel de chitosane, d'un gel de poly(N-isopropylacrylamide), d'un gel d'acide acrylique, d'un gel d'acide sulfonique de polystyrène, PPEGDA, PPEGDM, de polyacrylamide, de poly (N, N-diméthylacrylamide), de poly (N-isopropylacrylamide), DN hydrogel et un gel composite de deux ou plusieurs de ceux-ci et dans lequel un rapport de teneur en eau du matériau composite revêtu est de 20 % ou moins en poids ou dans lequel le matériau composite revêtu est plié à l'arrière.

2. Matériau composite revêtu selon la revendication 1, dans lequel le composé soluble sensible au pH est un composé soluble faiblement acide ou neutre, un composé soluble acide ou un composé soluble alcalin.

3. Matériau composite revêtu selon une revendication 1 ou 2, dans lequel le composé hydrosoluble est choisi dans un groupe constitué d'amidon, de polyéthylène glycol, de glycérogélatine, de pullulan, de pectine, de carraghénane, de β-glucane, de polymère à base d'ester de vinyle, de polymère à base d'ester vinylique, d'acide alginique, de dérivé d'acide alginique, de collagène, d'hydrolysat de collagène, de furcellaran, de gomme de graines de silume, de gomme de casse, de gomme de fenugrec, de gomme de pépins de coing, de gomme de tarabine, de gomme d'amande, de gomme de damson, arabinogalactan, dextran, de gomme rhamsan, levan, de gomme azotobacter vinelandii, de gomme enterobacter, de gomme welan, et une combinaison de deux ou plusieurs de celles-ci, le composé soluble sensible à la température est choisi parmi un composé constitué d'un groupe de gélatine, de graisse dure, de witepsol, de beurre de cacao, d'alcool polyvinylique, et une combinaison de deux ou plusieurs de celles-ci, le composé soluble sensible au pH est le composé soluble faiblement acide ou neutre et choisi parmi un groupe constitué de citrate d'acétyle et de tributyle, de carbomère, d'acétate de cellulose phtalate, d'acétate de cellulose succinate, d'acétate d'hypromellose succinate, d'hypromellose phtalate, d'acétate de polyvinyle phtalate, de gomme laque, de citrate de tributyle, de citrate de triéthyle, de cire blanche, de zéine, de succinate d'acétate d'hydroxypropylméthylcellulose, de phtalate d'acétate de polyvinyle, de phtalate d'hydroxypropylméthylcellulose, de gliadine de blé, de phtalate d'hypromellose, et une combinaison de deux ou plusieurs de ceux-ci, le composé soluble sensible au pH est le composé soluble acide et est choisi dans un groupe constitué de copolymère(s) E d'amino-méthacrylate d'alkyle, de diéthylaminoacétate d'acétal de polyvinyle, de chitosane, de copolymère(s) d'acide méthacrylique, d'éthylcellulose et d'une combinaison de deux ou plusieurs de ceux-ci, et le composé soluble sensible au pH est le composé soluble alcalin et est le copolymère d'acrylate d'éthyle et de méthacrylate de méthyle.

4. Matériau composite revêtu selon l'une quelconque des revendications 1 à 3, comprenant des particules exothermiques.

5. Matériau composite revêtu selon l'une quelconque des revendications 1 à 4, dans lequel le matériau composite est une électrode composite.

6. Matériau composite revêtu selon l'une quelconque des revendications 1 à 5, dans lequel le corps poreux est lié au substrat par un polymère s'étendant du substrat au corps poreux.

7. Matériau composite revêtu selon la revendication 6, dans lequel le polymère est au moins un polymère conducteur d'électricité choisi dans un groupe constitué de poly(3,4-éthylène-dioxythiophène), de polyacétylène, de polypyrrole, de polythiophène, de polybithiophène, de polyisothiophène, de polydodécylthiophène, de polyisonaphthiophène, de poly(3-hexylthiophène), de polyaniline, de polyisothianaphtène, de polythiazyle, de polyphénylène, de polyfluorène, de polydiacétylène, de polyacène, de polyparaphénylène, de polythénylène vinylène et de polyphénylènesulfure, ou un polymère isolant choisi dans un groupe constitué d'acide poly(méth)acrylique ou un de ses sels (par exemple, les acides poly(méth)acryliques tels que l'acide polyacrylique, l'acide polyméthacrylique, l'acide acrylique/alkylméthacrylique, l'acrylate de méthyle, l'acrylate de méthyle, l'acrylate de méthyle, l'acrylate de méthyle, l'acrylate de méthyle, l'acrylate de méthyle, etc, un copolymère d'acrylate/méthacrylate d'alkyle ou l'un de ses sels), de polymère de diméthacrylate de polyéthylène glycol (par exemple, PPEGDA ou PPEGDM), de polyhydroxyéthylméthacrylate, de polyacrylamide, de poly(N, N-diméthylacrylamide), de poly(2-acrylamido-2-méthylpropanesulfonique), de poly (N-isopropylacrylamide), de polyvinylpyrrolidone, de poly(acide styrène sulfonique), de polymère de carboxyvinyle, de polymère de carboxyvinyle modifié par des alkyles, de copolymère d'anhydride maléique, de résine d'oxyde de polyalkylène, de produit réticulé de poly(méthyl vinyl éther-alt-anhydride maléique) et de polyéthylène glycol, de produit réticulé de polyéthylène glycol, de produit réticulé de N-vinylacétamide, de produit réticulé d'acrylamide, de produit réticulé de copolymère greffé de sel d'amidon et d'acide acrylique et silicone.

8. Procédé de production d'un matériau composite revêtu selon l'une quelconque des revendications 1 à 7, le procédé comprenant les étapes consistant à :
(1) sécher un matériau composite dans lequel un corps poreux est lié à un substrat, et
(2) revêtir le matériau composite séché d'au moins un composant soluble choisi dans un groupe composé d'un composé soluble dans l'eau, d'un composé soluble sensible à la température et d'un composé soluble sensible au pH.

9. Procédé de production d'un matériau composite revêtu selon l'une quelconque des revendications 1 à 7, le procédé comprenant les étapes consistant à :
(1) revêtir un matériau composite dans lequel un corps poreux est lié à un substrat, avec au moins un composant soluble choisi dans un groupe constitué d'un composé soluble dans l'eau, d'un composé soluble sensible à la température et d'un composé soluble sensible au pH, et
(2) sécher le matériau composite revêtu.

10. Procédé de production d'un matériau composite revêtu selon l'une quelconque des revendications 1 à 7, le procédé comprenant les étapes consistant à :
(1) replier un matériau composite dans lequel un corps poreux est lié à un substrat, et
(2) revêtir le matériau composite plié d'au moins un composant soluble choisi dans un groupe composé d'un composé soluble dans l'eau, d'un composé soluble sensible à la température et d'un composé soluble sensible au pH.

11. Procédé de préparation d'un matériau composite revêtu selon l'une quelconque des revendications 8 à 10, dans lequel le matériau composite revêtu comprend des particules exothermiques.

12. Procédé de préparation d'un matériau composite revêtu selon l'une quelconque des revendications 8 à 11, dans lequel le composé hydrosoluble est choisi dans un groupe constitué d'amidon, de polyéthylène glycol, de glycérogélatine, de pullulan, de pectine, de carraghénane, de β-glucane, de polymère à base d'ester vinylique, d'acide alginique, de dérivé d'acide alginique, de collagène, d'hydrolysat de collagène, de furcellaran, de gomme de graines de silume, de gomme de casse, de gomme de fenugrec, de gomme de graines de coing, de gomme de tarabine, de gomme d'amande, de gomme de damson, arabinogalactan, dextran, de gomme rhamsan, de levan, de gomme azotobacter vinelandii, de gomme enterobacter, de gomme welan, et une combinaison de deux ou plusieurs de celles-ci, le composé soluble sensible à la température est choisi dans un composé constitué d'un groupe de gélatine, de graisse dure, de witepsol, de beurre de cacao, d'alcool polyvinylique, et une combinaison de deux ou plusieurs de ceux-ci, le composé soluble sensible au pH est le composé soluble faiblement acide ou neutre et choisi dans un groupe constitué de citrate d'acétyle et de tributyle, de carbomère, d'acétate de cellulose phtalate, d'acétate de cellulose succinate, d'acétate d'hypromellose succinate, d'hypromellose phtalate, d'acétate de polyvinyle phtalate, de gomme laque, de citrate de tributyle, de citrate de triéthyle, de cire blanche, de zéine, de succinate d'acétate d'hydroxypropylméthylcellulose, de phtalate d'acétate de polyvinyle, de phtalate d'hydroxypropylméthylcellulose, de gliadine de blé, de phtalate d'hypromellose, et une combinaison de deux ou plusieurs de ceux-ci, le composé soluble sensible au pH est le composé soluble acide et est choisi dans un groupe constitué de copolymère(s) E d'amino-méthacrylate d'alkyle, de diéthylaminoacétate d'acétal de polyvinyle, de chitosane, de copolymère(s) d'acide méthacrylique, d'éthylcellulose et d'une combinaison de deux ou plusieurs de ceux-ci, et le composé soluble sensible au pH est le composé soluble alcalin et est le copolymère d'acrylate d'éthyle et de méthacrylate de méthyle.

13. Procédé de préparation d'un matériau composite revêtu selon l'une quelconque des revendications 8 à 12, dans lequel le matériau composite revêtu est une électrode composite revêtue.

14. Procédé de préparation d'un matériau composite revêtu selon l'une quelconque des revendications 8 à 13, dans lequel le corps poreux est lié au substrat par polymérisation de monomères du substrat au corps poreux.
